## Europäisches Patentamt
## European Patent Office
## Office européen des brevets

(11) Publication number: **0 038 641**
**B1**

(19)

# EUROPEAN PATENT SPECIFICATION

(12)

(45) Date of publication of patent specification: **04.04.84**

(21) Application number: **81301469.3**

(22) Date of filing: **03.04.81**

(51) Int. Cl.³: **C 07 C 101/04,**
**C 07 C 99/00 //C07D501/18**

(54) **Process for producing D-2-amino-2-(1,4-cyclohexadienyl) acetic acid.**

(30) Priority: **21.04.80 JP 52590/80**

(43) Date of publication of application:
**28.10.81 Bulletin 81/43**

(45) Publication of the grant of the patent:
**04.04.84 Bulletin 84/14**

(84) Designated Contracting States:
**CH DE FR GB IT LI NL**

(56) References cited:
**FR - A - 1 445 767**
**FR - B - 2 173 636**
**US - A - 2 182 242**
**US - A - 3 682 981**

**Chemical Abstracts vol. 85, no. 23, 6 December 1976 Columbus, Ohio, USA "D-2-(1,4-Cyclohexadienyl)glycine" page 578, abstract no. 177985f**
**Chemical Abstracts vol. 78, no. 23, 11 June 1973 Columbus, Ohio, USA ASAHARA et al. "Reduction of Naphthalene in hexamethylphosphoric triamide-alkali metal-proton donor systems" page 351, column 2, abstract no. 147629f**

(73) Proprietor: **NIPPON KASEI CHEMICAL CO., LTD.**
**34 Onahama-Aza-Takayama**
**Iwaki-shi Fukushima-ken (JP)**

(72) Inventor: **Unuma, Kunio**
**6-9 Taira-Aza-Kufunjimachi**
**Iwaka-shi Fukushima-ken (JP)**
Inventor: **Saito, Hiroyasu**
**7-1 Aza-Kitaaramaki Hisanohamamachi**
**Iwaki-shi Fukushima-ken (JP)**
Inventor: **Inomata, Jihei**
**150 Onahama-Aza-Hayato**
**Iwaki-shi Fukushima-ken (JP)**
Inventor: **Takizawa, Saburo**
**3-6 Ueda 4-chome**
**Morioka-shi Iwate-ken (JP)**

(74) Representative: **Myerscough, Philip Boyd et al, J.A.Kemp & Co. 14, South Square Gray's Inn London, WC1R 5EU (GB)**

Courier Press, Leamington Spa, England.

### Process for producing D-2-amino-2-(1,4-cyclohexadienyl) acetic acid

The present invention relates to a process for producing D - 2 - amino - 2 - (1,4 - cyclohexadienyl)acetic acid by reducing D - 2 - aminophenyl - acetic acid with metallic sodium and a proton donor in the presence of liquid ammonia.

D-2-Amino-2-(1,4-cyclohexadienyl)acetic acid is useful as an intermediate compound for producing 6-aminopenicillanic acid or 7-aminocephalosporanic acid and has been prepared by reducing D-2-aminophenylacetic acid either with metallic lithium and t-butyl alcohol in liquid ammonia (U.S. Patent 3,682,981) or with metallic sodium and an alcohol in liquid ammonia (Spanish Patent No. 419,123). However, metallic lithium is expensive so that the process of U.S. Patent 3682981 is uneconomic, while in the process disclosed in Spanish Patent 419,123 the rate of reduction is low. Moreover, in these processes an alcohol is used as proton donor and must be recovered industrially after the reaction is over. In one recovery procedure, after decomposing the alcoholate of metallic lithium or metallic sodium, which has formed in the reaction system, using triethylamine hydrochloride, ammonia in the reaction system is evaporated off, and the unreacted D-2-aminophenylacetic acid and the reaction product, D-2-amino-2-(1,4-cyclohexadienyl)acetic acid, which remain in the system slurried with the alcohol, are heated to evaporate the alcohol. Such a recovery procedure can cause racemization of the D-2-aminophenylacetic acid and D - 2 - amino - 2 - (1,4 - cyclohexadienyl)acetic acid unless the alcohol is evaporated under a reduced pressure at a relatively low temperature, but then, D-2-aminophenylacetic acid and D-2-amino-2-(1,4-cyclohexadienyl)acetic acid are entrained by the evaporating alcohol and effective recovery of the alcohol is difficult.

The present invention provides a process for effectively producing D-2-amino-2-(1,4-cyclohexadienyl)acetic acid from D-2-aminophenylacetic acid without using any alcohol.

According to the process of the present invention D-2-amino-2-(1,4-cyclohexadienyl)acetic acid is produced by reducing D-2-aminophenylacetic acid with metallic sodium and a proton donor in the presence of liquid ammonia characterized in that water is used as proton donor, the weight ratio of liquid ammonia to water is from 65:35 to 82:18, from 3 to 5.5 kg of a mixture of water and liquid ammonia and more than one mole of metallic sodium are used per mole of D-2-aminophenylacetic acid, and the reduction is carried out at a temperature of from −25°C to −50°C.

The ratio of liquid ammonia to water in the mixture is from 65:35 to 82:18 and this permits the easy removal of unreacted substance from the reaction system.

If water alone was used as the reaction medium, there would be a danger of explosion during the reaction which accordingly could not be carried out safely.

The amount of the mixture of liquid ammonia and water for use in the process is 3 to 5.5 kg per mole of the starting material, D-2-aminophenylacetic acid, and in general the smaller the amount of the mixture the slower is the rate of reduction, while the greater the amount the smaller is the feed amount of the starting material and the longer is the period required to recover liquid ammonia after the reaction is over.

The metallic sodium used in the process of the invention reacts with water in the reaction medium to generate hydrogen which contributes to the reduction of D-2-aminophenylacetic acid while forming sodium hydroxide which reacts with the thus formed D-2-amino-2-(1,4-cyclohexadienyl)acetic acid and unreacted D-2-aminophenylacetic acid to convert them into their sodium salts. Accordingly, metallic sodium being used in an amount of more than the equimolar amount relative to the starting material, D-2-aminophenylacetic acid, unreacted starting material and the reaction product, D - 2 - amino - 2 - (1,4 - cyclohexadienyl)acetic acid, are obtained as their sodium salts dissolved in water after the reaction is over. Therefore, by adding an acidic substance, such as sulfuric or hydrochloric acid, to the solution of the salts, D-2-aminophenylacetic acid or D-2-amino-2-(1,4-cyclohexadienyl)acetic acid separate out from the aqueous solution and can be easily recovered.

In consideration of the foregoing, metallic sodium is used in an amount of more than an equimolar amount relative to the starting material, preferably in a range of 3 to 10 mole per mole of D-2-aminophenylacetic acid.

Although it is necessary to maintain the reaction temperature of the process of the present invention sufficiently low as to prevent racemization of the optically active D-2-aminophenylacetic acid and the optically active D - 2 - amino - 2 - (1,4 - cyclohexadienyl)acetic acid, when the reaction temperature is too low, the rate of dissolution of metallic sodium into the reaction medium is reduced and with it the reaction rate. Accordingly the reaction temperature should be maintained at from −50 to −25°C. The reaction is usually complete (as confirmed by the complete consumption of metallic sodium introduced into the reaction system) within about 30 minutes at these temperatures.

In carrying out the process of the present invention, D-2-aminophenylacetic acid and water are introduced into a reaction vessel, and then sufficient liquid ammonia is added to the system to provide the desired weight ratio of liquid ammonia to water under agitation while maintaining the desired temperature of the system to dissolve the starting material in the reaction medium, and an amount of metallic sodium more than the equimolar amount relative to the D-2-aminophenylacetic acid is added. Upon dissolving metallic sodium in the system containing the reactants, the reaction mixture colours

**0 038 641**

blue and bubbles of gaseous hydrogen are formed. As the reaction proceeds the colour of the reaction mixture changes from blue to white and a complete change of colour to white indicates completion of the reaction.

When the reaction is complete, the temperature within the reaction vessel is gradually raised to evaporate liquid ammonia and the ammonia is recovered for re-use. The remaining reaction mixture is filtered to remove water-insoluble matter and to provide a clear aqueous solution as the filtrate. On adjusting the pH of the aqueous solution by adding an acidic substance, such as sulfuric or hydrochloric acid, crude D-2-amino-2-(1,4-cyclohexadienyl)acetic acid is obtained as white crystals.

As has been described, by controlling the weight ratio of liquid ammonia to water in the reaction medium, the weight ratio of the reaction medium to the starting material and the reaction temperature, D - 2 - amino - 2 - (1,4 - cyclohexadienyl)acetic acid is effectively produced from D-2-aminophenylacetic acid without using any alcohol.

The present invention will be illustrated by the following examples:

Examples 1 to 7 and Comparative Examples 1 to 3

In a 5-litre round-bottomed glass flask provided with a stirrer, a thermometer, an inlet for metallic sodium and an outlet for gaseous ammonia and gaseous hydrogen to the ambient atmosphere, 100 g (0.66 mol) of D-2-aminophenylacetic acid showing a specific rotatory power of $[\alpha]_D^{25} = -110°$ and water in the amount shown in the Table which follows were cooled to −35°C.

An amount of liquid ammonia as shown in the Table was added to the cooled mixture and the contents of the flask were stirred to dissolve the D-2-aminophenylacetic acid. Then 92 g (3.91 mols) of small pieces of metallic sodium were added over 30 minutes while controlling the rate of addition so as not to raise the temperature of the reaction mixture to higher than −25°C. The metallic sodium gradually dissolved in the reaction mixture colouring the reaction mixture blue and causing the incessant evolution of gaseous hydrogen.

10 Minutes after completing the addition of metallic sodium, the colour of the reaction mixture changed from blue to white indicating that the metallic sodium had been completely consumed, then the temperature of reaction mixture was gradually raised to evaporate the liquid ammonia, and the thus obtained sludge of reaction mixture was subjected to filtration to collect a clear aqueous solution.

While cooling the aqueous solution, 35% hydrochloric acid was added to bring its pH to 6.5, and the thus precipitated white crystals were collected by filtration.

After washing the thus obtained crystals three times with de-ionized water, they were dried under a reduced pressure. By analyzing the thus obtained product by nuclear magnetic resonance spectroscopy following the method described in J. Pharmaceut. Sci., Vol. 65(5), pages 738—740 (1976) and determining its specific rotatory power, in 2N NaOH aq. at 1% concentration it was confirmed that the product was crude D - 2 - amino - 2 - (1,4 - cyclohexadienyl)acetic acid containing residual starting material, D-2-aminophenylacetic acid.

The thus obtained values of its specific rotatory power, its weight (including the starting material) and its yield are shown in the Table.

The yield was obtained by gas chromatographic analysis of the product after subjecting the crude product to trimethylsilylization with bis-(trimethylsilyl) trifluoroacetamide.

3

# 0 038 641

TABLE

| | Amount of reaction medium (g) | | Specific rotatory power $[\alpha]_D^{26}(°)$ | Weight of product (g) | Yield (%)** |
|---|---|---|---|---|---|
| | liq. NH₃* | water | | | |
| Comp Ex 1 | 1860 | 140 | −116 | 94.1 | 8.6 |
| Comp Ex 2 | 1740 | 260 | −115 | 93.9 | 35.3 |
| Example 1 | 1640 | 360 | −115 | 93.5 | 68.0 |
| Example 2 | 1540 | 460 | −120 | 93.0 | 83.3 |
| Example 3 | 1460 | 540 | −118 | 92.6 | 84.7 |
| Example 4 | 1420 | 580 | −115 | 92.5 | 82.5 |
| Example 5 | 1320 | 680 | −117 | 92.2 | 66.0 |
| Comp Ex 3 | 1260 | 740 | −116 | 91.8 | 46.7 |
| Example 6 | 2500 | 1000 | −118 | 90.5 | 95.1 |
| Example 7 | 2660 | 840 | −116 | 91.0 | 96.9 |

Note: * liquid ammonia.
      ** vs. theoretical value.

**Claim**

A process for producing D-2-amino-2-(1,4-cyclohexadienyl)acetic acid by reducing D-2-aminophenylacetic acid with metallic sodium and a proton donor in the presence of liquid ammonia characterized in that water is used as proton donor, the weight ratio of liquid ammonia to water is from 65:35 to 82:18, from 3 to 5.5 kg of a mixture of water and liquid ammonia and more than one mole of metallic sodium are used per mole of D-2-aminophenylacetic acid, and the reduction is carried out at a temperature of from −25°C to −50°C.

**Revendication**

Un procédé pour la production d'acide D-2-amino-2-(1,4-cyclohexadiényle)acétique par réduction de l'acide D-2-aminophénylacétique avec du sodium métallique et un donneur de proton en présence d'ammoniac liquide, caractérisé en ce qu'on utilise l'eau comme donneur de proton, le rapport pondéral de l'ammoniac liquide à l'eau est de 65/35 à 82/18, on utilise de 3 à 5,5 kg d'un mélange d'eau et d'ammoniac liquide et plus d'une mole de sodium métallique par mole d'acide D-2-aminophénylacétique, et on effectue la réduction à une température de −25°C à −50°C.

**Patentanspruch**

Verfahren zur Herstellung von D-2-Amino-2-(1,4-cyclohexadienyl)essigsäure durch Reduktion von D-2-Aminophenylessigsäure mit metallischem Natrium und einem Protonendonor in Gegenwart von flüssigem Ammoniak, dadurch gekennzeichnet, daß als Protonendonor Wasser verwendet wird, das Gewichtsverhältnis von flüssigem Ammoniak zu Wasser von 65:35 bis 82:18 beträgt, pro Mol der D-2-Aminophenylessigsäure von 3 bis 5,5 kg einer Mischung aus Wasser und flüssigem Ammoniak und mehr als ein Mol metallisches Natrium verwendet werden, und die Reduktion bei einer Temperatur von −25°C bis −50°C durchgeführt wird.